(19) **Europäisches Patentamt / European Patent Office / Office européen des brevets**

(11) **EP 3 313 092 A1**

(12) **EUROPEAN PATENT APPLICATION**

(43) Date of publication:
**25.04.2018 Bulletin 2018/17**

(21) Application number: **17161519.8**

(22) Date of filing: **17.03.2017**

(51) Int Cl.:
**H04R 25/00** (2006.01)   **A61B 5/04** (2006.01)
**A61B 5/00** (2006.01)   A61B 5/1455 (2006.01)
**H04R 5/033** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Oticon A/S
2765 Smørum (DK)**

(72) Inventors:
• **LAPLANTE-LÉVESQUE, Ariane
DK-2765 Smørum (DK)**
• **BJELOSEVIC, Adis
DK-2765 Smørum (DK)**

(74) Representative: **William Demant
Oticon A/S
Kongebakken 9
2765 Smørum (DK)**

(54) **A HEARING SYSTEM FOR MONITORING A HEALTH RELATED PARAMETER**

(57)    A binaural hearing system comprises a) left and right hearing devices, e.g. hearing aids, adapted for being worn at or in left and right ears, respectively, of a user, or for being fully or partially implanted in the head at the left and right ears, respectively, of the user, each of the left and right hearing devices comprising a1) a number Ns of different sensors Si (i=1, ..., Ns), each sensor being configured to monitor a physiological function of the user and providing respective left and right sensor signals $ST_{i,left}$, $ST_{i,right}$ (i=1, ..., Ns), indicative of the state of the physiological function in question; a2) antenna and transceiver circuitry to provide that information signals, including said sensor signals can be exchanged between the left and right hearing devices and/or forwarded to an auxiliary device, b) a comparison unit for comparing said left and right sensor signals, and providing respective comparison signals $CSI_i$ (i=1, ..., Ns) for each of said physiological functions; and c) an analysis unit for analyzing said comparison signals and providing a concluding stroke indicator CSI regarding a risk of stroke of the user depending on said comparison signal(s). Thereby an improved functionality of a hearing system may be provided allowing an early warning of a stroke of a wearer of the hearing system.

A method of detecting a risk of stroke of a user wearing a binaural hearing system comprising left and right hearing devices, the method comprising

FIG. 1

EP 3 313 092 A1

**Description**

SUMMARY

The present application relates to the field of hearing devices, e.g. hearing aids, and in particular to a hearing system comprising left and right hearing devices for wear on left and right sides of the head of a user, and comprising sensors for monitoring health related parameters of the user.

A hearing device:

In an aspect of the present application, a binaural hearing system is provided. The binaural hearing system comprises

- left and right hearing devices, e.g. hearing aids, adapted for being worn at or in left and right ears, respectively, of a user, or for being fully or partially implanted in the head at the left and right ears, respectively, of the user, each of the left and right hearing devices comprising

  ○ A number $N_S$ of different sensors $S_i$ (i=1, ..., $N_S$), each sensor being configured to monitor a physiological function of the user and providing respective left and right sensor signals $ST_{i,left}$, $ST_{i,right}$ (i=1, ..., $N_S$), indicative of the state of the physiological function in question;
  ○ Antenna and transceiver circuitry to provide that information signals, including said sensor signals $ST_{i,left}$, $ST_{i,right}$ (i=1, ..., $N_S$), or parts thereof or data originating therefrom, can be exchanged between the left and right hearing devices and/or forwarded to an auxiliary device.

The hearing system further comprises,

- A comparison unit for comparing said left and right sensor signals $ST_{i,left}$, $ST_{i,right}$ (i=1, ..., Ns), or parts thereof, or data originating therefrom, and providing respective comparison signals $CSI_i$ (i=1, ..., $N_S$) for each of said physiological functions; and
- An analysis unit for analyzing said comparison signals $CSI_i$ (i=1, ..., $N_S$) and providing a concluding stroke indicator CSI regarding a risk of stroke of the user depending on said comparison signal(s).

Thereby an improved functionality of a hearing device may be provided.
In an embodiment, the number of sensors $N_S$ is one or more. In an embodiment, the number of sensors $N_S$ is two or more. In an embodiment, a sensor stroke indicator $SSI_i$ (i=1, ..., $N_S$) is determined for each sensor based on the left and right sensor signals $ST_{i,left}$, $ST_{i,right}$ for the sensor in question, e.g. based on said comparison signals $CSI_i$ (i=1, ..., Ns). In an embodiment, the sensor stroke indicator $SSI_i$ (i=1, ..., $N_S$) is based on a distance measure between the left and right sensor signals $ST_{i,left}$, $ST_{i,right}$. In an embodiment, the distance measure is given by a mathematical distance measure (e.g. a distance function or metric, such as an Euclidian distance) or a statistical distance measure (such as an energy distance or a power distance).
In an embodiment, the binaural hearing system comprises an alarm unit configured to initiate the issue of an alarm in case said concluding stroke indicator CSI indicates a risk of stroke of the user larger than a threshold value. In an embodiment, the concluding stroke indicator CSI takes on values between 0 and 1. In an embodiment, a value of the concluding stroke indicator CSI of less than 0.20 indicates a low estimated risk and a value larger than 0.8 indicates a high estimated risk. In an embodiment, the alarm unit is configured to initiate the issue of an alarm in case said concluding stroke indicator CSI has a value larger than 0.8.
In an embodiment, at least one of the number of sensors comprises an electrode for picking up electric signals of the body. In an embodiment, signals from the body represent signals from the user's head. In an embodiment, signals from the body represent evoked potentials (e.g. electrically or acoustically or optically evoked potentials). In an embodiment, signals from the body represent signals from the user's brain or associated nerves. In an embodiment, signals from the body represent signals from muscular activity. In an embodiment, signals from the body represent signals from the eyes (e.g. EOG potentials).
In an embodiment, at least one of the sensors is configured to measure brain activity, e.g. to pick up signals from the user's brain, e.g. EEG-potentials. In an embodiment, a sensor stroke indicator $SSI_{EEG}$ providing a measure of the amount of ischemic damage of the brain is provided by a brain symmetry index (BSI) as defined by the following expression (cf. e.g. [van Putten & Tavy; 2012]):

$$SS_{EEG} = BSI(t) \frac{1}{M} \sum_{j=1}^{M} \left\| \sum_{i=1}^{N} \frac{R_{ij}(t) - L_{ij}(t)}{R_{ij}(t) + L_{ij}(t)} \right\|$$

where $t$ is time, $N$ is the number of EEG-channel pairs, and M is the number of Fourier coefficients. The BSI represents the mean of the absolute value of the difference in mean power (e.g. defined by the power spectral density) between the respective left and right EEG channels in the frequency range from 1 Hz to 25 Hz. In an embodiment, the power spectral density is estimated by fast Fourier transform, in which case the power of the signal obtained from a particular bipolar channel pair $i$ (with $i$=1, 2, ..., $N$) at frequency $j$ (or Fourier coefficient, with index $j$=1, 2, ..., $M$), $R_{ij}(t)$ and $L_{ij}(t)$ for the right and left sensors, respectively. In an embodiment, the left and right sensor signals $ST_{EEG,left}(i,j)$, $ST_{EEG,right}(i,j)$ is represented by the signal powers $L_{ij}(t)$ and $R_{ij}(t)$, respectively. For a hearing device comprising EEG electrodes located at or in the ear, $N$ is typically less than or equal to 5, e.g. from 2-4, such as equal to 1. In an embodiment, the number of Fourier coefficients is less than 1024, e.g. less than 512. In an embodiment, the brain activity sensor comprises an analogue to digital (AD) converter. In an embodiment, a sampling frequency $f_s$ of the AD converter for digitizing the EEG signals is larger than 50 Hz, e.g. larger than 1 kHz, e.g. in the range from 50 Hz to 200 Hz. In an embodiment, the BSI is provided every time frame of the left and right sensor signals. In an embodiment, the BSI is provided with a frequency higher than 1 Hz. In an embodiment, the BSI is provided with a frequency lower than 1 Hz, e.g. lower than or equal to 0.2 Hz. The lower bound for the BSI is zero (which implies perfect symmetry for all channels), whereas the upper bound is 1 (which implies maximal asymmetry). For healthy controls, the BSI is 0.042 +/-0.005 (based on a digital EEG database [van Putten & Tavy; 2012]).

In an embodiment, at least one of said sensors is configured to measure ocular muscle activity, e.g. using electrooculography (EOG). Ocular muscle activity may e.g. be observed as artifacts in an EEG signal from the brain e.g. in the form of spikes of (much) larger amplitude than the brain wave signals, e.g. more localized in time, and/or occurring another frequency than brain wave signals. In an embodiment, a sensor stroke indicator $SSI_{EOG}$ providing a measure of the difference between the detected ocular muscle activity of the left and right ocular muscle activity sensors, e.g. EOG-sensors, is provided.

In an embodiment, EEG or EOG data are digitized and/or amplified, e.g. by an analogue to digital converter. In an embodiment, EEG or EOG data are post-processed to remove drift and artefacts, e.g. by Kalman filtering.

In an embodiment, at least one of said number of sensors is configured to measure oxygen saturation of the user's blood, e.g. using pulse oximetry. A system, e.g. comprising a hearing aid, comprising a blood sensor, e.g. for measuring oxygen in the blood (or blood sugar or blood pressure), e.g. in the form of a pulse oximeter, is described in our co-pending European patent application no. 16162760.9, published as EP3035710A2.

In an embodiment, at least one of said number of sensors is configured to monitor jaw activity or neck muscle activity. In an embodiment, at least one of said number of sensors is configured to monitor activity in the temporomandibular joint (TMJ). In an embodiment, the sensor configured to monitor TMJ activity or neck muscle activity comprises a radar sensor. A hearing aid comprising a remote object detection unit configured to emit an electromagnetic signal and to detect a remote object by detecting an electromagnetic signal reflected off the object, e.g. a radar sensor, is described in US20150319542A1.

In an embodiment, the number $N_S$ of different sensors $S_i$ ($i$=1, ..., $N_S$) comprises a first sensor comprising an electrode for picking up electric signals of the body, e.g. EEG signals and/or EOG signals, and at least one additional sensor. In an embodiment, sensor stroke indicators $SSI_{EEG}$ and $SSI_{other}$ are determined based on respective left and right sensor signals ($ST_{EEG,left}$, $ST_{EEG,right}$) and ($ST_{other,left}$, $ST_{other,right}$), respectively, e.g. based on respective comparison signals $CSI_{EEG}$ and $CSI_{other}$, respectively. In an embodiment, the concluding stroke indicator CSI is based on a predefined conditional criterion regarding the comparison signals $CSI_i$ or sensor stroke indicator $SSI_i$ ($i$=1, ..., $N_S$) of the first sensor and said at least one additional sensor in such a way that the comparison signal or the sensor stroke indicator of the at least one additional sensor is only considered in case a first comparison signal $CSI_{EEG}$ or a first sensor stroke indicator $SSI_{EEG}$ is indicative of a stroke.

In an embodiment, the predefined criterion regarding the resulting status indicators comprises a degree of asymmetry of the left and right status signals $ST_{i,left}$, $ST_{i,right}$ ($i$=1, ..., N) as reflected in the corresponding comparison signals $CSI_i$ ($i$=1, ..., N). In an embodiment, the degree of asymmetry is determined by a distance measure indicative of a difference between the left and right status signals $ST_{i,left}$, $ST_{i,right}$ ($i$=1, ..., N).

In an embodiment, the sensor stroke indicator(s) and/or the conclusive stroke indicator is/are determined using machine learning techniques, such as artificial intelligence, big data, and/or neural networks (e.g. deep neural networks) in the data analysis.

In an embodiment, at least one of said number of sensors is configured to measure L/R Vestibulo-ocular reflex (VOR) asymmetry, e.g. based on Video-oculography, for early detection of stroke.

In an embodiment, the binaural hearing system comprises a wireless interface allowing the concluding stroke indicator and/or the alarm to be forwarded to another device, e.g. via a network. In an embodiment, the binaural hearing system is configured to transmit information about a risk of an ischemic stroke according to predefined contact data, e.g. a telephone number or an IP-address or an URL.

In an embodiment, the left and right hearing devices comprises left and right hearing aids, headsets, earphones, ear protection devices or a combination thereof.

In an embodiment, the left and right hearing devices form part of or are mechanically and/or electrically connected to glasses, a head band, a cap, or any other carrier adapted for being located on the head of the user.

In a further aspect, a hearing system as described above, in the 'detailed description of embodiments', and in the claims, AND an auxiliary device is moreover provided.

In an embodiment, the system is adapted to establish a communication link between the hearing device and the auxiliary device to provide that information (e.g. control and status signals, possibly audio signals) can be exchanged or forwarded from one to the other.

In an embodiment, the auxiliary device is or comprises an audio gateway device adapted for receiving a multitude of audio signals (e.g. from an entertainment device, e.g. a TV or a music player, a telephone apparatus, e.g. a mobile telephone or a computer, e.g. a PC) and adapted for selecting and/or combining an appropriate one of the received audio signals (or combination of signals) for transmission to the hearing device. In an embodiment, the auxiliary device is or comprises a remote control for controlling functionality and operation of the hearing device(s). In an embodiment, the function of a remote control is implemented in a SmartPhone, the SmartPhone possibly running an APP allowing to control the functionality of the audio processing device via the SmartPhone (the hearing device(s) comprising an appropriate wireless interface to the SmartPhone, e.g. based on Bluetooth or some other standardized or proprietary scheme).

In the present context, a SmartPhone, may comprise

- a (A) cellular telephone comprising a microphone, a speaker, and a (wireless) interface to the public switched telephone network (PSTN) COMBINED with
- a (B) personal computer comprising a processor, a memory, an operative system (OS), a user interface (e.g. a keyboard and display, e.g. integrated in a touch sensitive display) and a wireless data interface (including a Web-browser), allowing a user to download and execute application programs (APPs) implementing specific functional features (e.g. displaying information retrieved from the Internet, remotely controlling another device, combining information from various sensors of the smartphone (e.g. camera, scanner, GPS, microphone, etc.) and/or external sensors to provide special features, etc.).

A hearing device:

In an embodiment, the hearing device is adapted to provide a frequency dependent gain and/or a level dependent compression and/or a transposition (with or without frequency compression) of one or frequency ranges to one or more other frequency ranges, e.g. to compensate for a hearing impairment of a user. In an embodiment, the hearing device comprises a signal processor for enhancing the input signals and providing a processed output signal.

In an embodiment, the hearing device comprises an output unit for providing a stimulus perceived by the user as an acoustic signal based on a processed electric signal. In an embodiment, the output unit comprises a number of electrodes of a cochlear implant or a vibrator of a bone conducting hearing device. In an embodiment, the output unit comprises an output transducer. In an embodiment, the output transducer comprises a receiver (loudspeaker) for providing the stimulus as an acoustic signal to the user. In an embodiment, the output transducer comprises a vibrator for providing the stimulus as mechanical vibration of a skull bone to the user (e.g. in a bone-attached or bone-anchored hearing device).

In an embodiment, the hearing device comprises an input unit for providing an electric input signal representing sound. In an embodiment, the input unit comprises an input transducer, e.g. a microphone, for converting an input sound to an electric input signal. In an embodiment, the input unit comprises a wireless receiver for receiving a wireless signal comprising sound and for providing an electric input signal representing said sound.

In an embodiment, the hearing device comprises a directional microphone system adapted to spatially filter sounds from the environment, and thereby enhance a target acoustic source among a multitude of acoustic sources in the local environment of the user wearing the hearing device. In an embodiment, the directional system is adapted to detect (such as adaptively detect) from which direction a particular part of the microphone signal originates. This can be achieved in various different ways as e.g. described in the prior art.

In an embodiment, the hearing device comprises an antenna and transceiver circuitry (e.g. a wireless receiver) for wirelessly receiving a direct electric input signal from another device, e.g. from an entertainment device (e.g. a TV-set), a communication device, a wireless microphone, or another hearing device. In an embodiment, the direct electric input signal represents or comprises an audio signal and/or a control signal and/or an information signal. In an embodiment, the hearing device comprises demodulation circuitry for demodulating the received direct electric input to provide the direct electric input signal representing an audio signal and/or a control signal e.g. for setting an operational parameter (e.g. volume) and/or a processing parameter of the hearing device. In general, a wireless link established by antenna and transceiver circuitry of the hearing device can be of any type. In an embodiment, the wireless link is established between two devices, e.g. between an entertainment device (e.g. a TV) and the hearing device, or between two hearing devices, e.g. via a third, intermediate device (e.g. a processing device, such as a remote control device, a smartphone, etc.). In an embodiment, the wireless link is used under power constraints, e.g. in that the hearing device is or comprises a portable (typically battery driven) device. In an embodiment, the wireless link is a link based on near-field communication, e.g. an inductive link based on an inductive coupling between antenna coils of transmitter and receiver parts. In another embodiment, the wireless link is based on far-field, electromagnetic radiation. In an embodiment, the communication via the wireless link is arranged according to a specific modulation scheme, e.g. an analogue modulation scheme, such as FM (frequency modulation) or AM (amplitude modulation) or PM (phase modulation), or a digital modulation scheme, such as ASK (amplitude shift keying), e.g. On-Off keying, FSK (frequency shift keying), PSK (phase shift keying), e.g. MSK (minimum shift keying), or QAM (quadrature amplitude modulation), etc.

In an embodiment, the communication between the hearing device and the other device is in the base band (audio frequency range, e.g. between 0 and 20 kHz). Preferably, communication between the hearing device and the other device is based on some sort of modulation at frequencies above 100 kHz. Preferably, frequencies used to establish a communication link between the hearing device and the other device is below 50 GHz, e.g. located in a range from 50 MHz to 70 GHz, e.g. above 300 MHz, e.g. in an ISM range above 300 MHz, e.g. in the 900 MHz range or in the 2.4 GHz range or in the 5.8 GHz range or in the 60 GHz range (ISM=Industrial, Scientific and Medical, such standardized ranges being e.g. defined by the International Telecommunication Union, ITU). In an embodiment, the wireless link is based on a standardized or proprietary technology. In an embodiment, the wireless link is based on Bluetooth technology (e.g. Bluetooth Low-Energy technology).

In an embodiment, the hearing device is portable device, e.g. a device comprising a local energy source, e.g. a battery, e.g. a rechargeable battery.

In an embodiment, the hearing device comprises a forward or signal path between an input unit (e.g. an input transducer, such as a microphone or a microphone system and/or direct electric input (e.g. a wireless receiver)) and an output unit, e.g. an output transducer. In an embodiment, the signal processor is located in the forward path. In an embodiment, the signal processor is adapted to provide a frequency dependent gain according to a user's particular needs. In an embodiment, the hearing device comprises an analysis path comprising functional components for analyzing the input signal (e.g. determining a level, a modulation, a type of signal, an acoustic feedback estimate, etc.). In an embodiment, some or all signal processing of the analysis path and/or the signal path is conducted in the frequency domain. In an embodiment, some or all signal processing of the analysis path and/or the signal path is conducted in the time domain.

In an embodiment, an analogue electric signal representing an acoustic signal is converted to a digital audio signal in an analogue-to-digital (AD) conversion process, where the analogue signal is sampled with a predefined sampling frequency or rate $f_s$, $f_s$ being e.g. in the range from 8 kHz to 48 kHz (adapted to the particular needs of the application) to provide digital samples $x_n$ (or $x[n]$) at discrete points in time $t_n$ (or $n$), each audio sample representing the value of the acoustic signal at $t_n$ by a predefined number $N_b$ of bits, $N_b$ being e.g. in the range from 1 to 48 bits, e.g. 24 bits. Each audio sample is hence quantized using $N_b$ bits (resulting in $2^{Nb}$ different possible values of the audio sample). A digital sample x has a length in time of $1/f_s$, e.g. 50 $\mu$s, for $f_s$ = 20 kHz. In an embodiment, a number of audio samples are arranged in a time frame. In an embodiment, a time frame comprises 64 or 128 audio data samples. Other frame lengths may be used depending on the practical application.

In an embodiment, the hearing devices comprise an analogue-to-digital (AD) converter to digitize an analogue audio input with a (first) predefined (first) sampling rate $f_{s1}$, e.g. $f_{s1} \geq$ 16 kHz. In an embodiment, the hearing devices comprise a digital-to-analogue (DA) converter to convert a digital signal to an analogue output signal, e.g. for being presented to a user via an output transducer. the hearing devices comprise an analogue-to-digital (AD) converter to digitize an analogue sensor input (e.g. from a sensor, such as a sensor for picking up electric-magnetic signals from the body of a user) with a (second) predefined (second) sampling rate $f_{s2}$, e.g. $f_{s2} \geq$ 50 Hz

In an embodiment, the hearing device, e.g. the microphone unit, and or the transceiver unit comprise(s) a TF-conversion unit for providing a time-frequency representation of an input signal. In an embodiment, the time-frequency representation comprises an array or map of corresponding complex or real values of the signal in question in a particular time and frequency range. In an embodiment, the TF conversion unit comprises a filter bank for filtering a (time varying) input signal and providing a number of (time varying) output signals each comprising a distinct frequency range of the input signal. In an embodiment, the TF conversion unit comprises a Fourier transformation unit for converting a time variant input signal to a (time variant) signal in the frequency domain. In an embodiment, the frequency range considered by the hearing device from a minimum frequency $f_{min}$ to a maximum frequency $f_{max}$ comprises a part of the typical human audible frequency range from 20 Hz to 20 kHz, e.g. a part of the range from 20 Hz to 12 kHz. In an embodiment, a signal of the forward and/or analysis path of the hearing device is split into a number *NI* of frequency bands, where NI is e.g.

larger than 5, such as larger than 10, such as larger than 50, such as larger than 100, such as larger than 500, at least some of which are processed individually. In an embodiment, the hearing device is/are adapted to process a signal of the forward and/or analysis path in a number *NP* of different frequency channels (*NP ≤ NI)*. The frequency channels may be uniform or non-uniform in width (e.g. increasing in width with frequency), overlapping or non-overlapping.

In an embodiment, the hearing system, e.g. the left and/or right hearing device, comprises a number of detectors configured to provide status signals relating to a current physical environment of the hearing device (e.g. the current acoustic environment), and/or to a current state of the user wearing the hearing device, and/or to a current state or mode of operation of the hearing device. Alternatively or additionally, one or more detectors may form part of an *external* device in communication (e.g. wirelessly) with the hearing device. An external device may e.g. comprise another hearing device, a remote control, and audio delivery device, a telephone (e.g. a Smartphone), an external sensor, etc.

In an embodiment, one or more of the number of detectors operate(s) on the full band signal (time domain). In an embodiment, one or more of the number of detectors operate(s) on band split signals ((time-) frequency domain).

In an embodiment, the number of detectors comprises a level detector for estimating a current level of a signal of the forward path. In an embodiment, the predefined criterion comprises whether the current level of a signal of the forward path is above or below a given (L-)threshold value. In an embodiment, the level detector operates on the full band signal (time domain). In an embodiment, the level detector operates on band split signals ((time-)frequency domain).

In a particular embodiment, the hearing device comprises a voice detector (VD) for determining whether or not an input signal comprises a voice signal (at a given point in time). A voice signal is in the present context taken to include a speech signal from a human being. It may also include other forms of utterances generated by the human speech system (e.g. singing). In an embodiment, the voice detector unit is adapted to classify a current acoustic environment of the user as a VOICE or NO-VOICE environment. This has the advantage that time segments of the electric microphone signal comprising human utterances (e.g. speech) in the user's environment can be identified, and thus separated from time segments only comprising other sound sources (e.g. artificially generated noise). In an embodiment, the voice detector is adapted to detect as a VOICE also the user's own voice. Alternatively, the voice detector is adapted to exclude a user's own voice from the detection of a VOICE.

In an embodiment, the hearing device comprises an own voice detector for detecting whether a given input sound (e.g. a voice) originates from the voice of the user of the system. In an embodiment, the microphone system of the hearing device is adapted to be able to differentiate between a user's own voice and another person's voice and possibly from NON-voice sounds.

In an embodiment, the number of detectors comprises a movement detector, e.g. an acceleration sensor. In an embodiment, the movement detector is configured to detect movement of the user's facial muscles and/or bones, e.g. due to speech or chewing (e.g. jaw movement) and to provide a detector signal indicative thereof.

In an embodiment, the hearing system, e.g. the left and/or right hearing device, comprises a temperature sensor, a light sensor, a time indicator, a magnetic field sensor, a humidity sensor, or any other sensor useful for estimating a health condition of the user.

In an embodiment, the hearing device comprises a classification unit configured to classify the current situation based on input signals from (at least some of) the detectors, and possibly other inputs as well. In the present context 'a current situation' is taken to be defined by one or more of

a) the physical environment (e.g. including the current electromagnetic environment, e.g. the occurrence of electromagnetic signals (e.g. comprising audio and/or control signals) intended or not intended for reception by the hearing device, or other properties of the current environment than acoustic;
b) the current acoustic situation (input level, feedback, etc.), and
c) the current mode or state of the user (movement, temperature, etc.);
d) the current mode or state of the hearing device (program selected, time elapsed since last

user interaction, etc.) and/or of another device in communication with the hearing device.

In an embodiment, the hearing device further comprises other relevant functionality for the application in question, e.g. feedback cancellation, compression, noise reduction, etc.

Use:

In an aspect, use of a hearing system as described above, in the 'detailed description of embodiments' and in the claims, is moreover provided. In an embodiment, use is provided in a system comprising one or more hearing instruments, headsets, ear phones, active ear protection systems, etc. In an embodiment, use for detecting an unilateral process influencing health of a user, e.g. functionality of the brain of the user, e.g. a risk of stroke of the user.

A method:

In an aspect, method of detecting a risk of stroke of a user wearing a binaural hearing system comprising left and right hearing devices, e.g. hearing aids, adapted for being worn at or in left and right ears, respectively, of a user, or for being fully or partially implanted in the head at the left and right ears, respectively, of the user is furthermore provided. The method comprises

- In each of the left and right hearing devices

  ○ providing respective left and right sensor signals $ST_{i,left}$, $ST_{i,right}$ (i=1, ..., Ns) indicative of a state of a physiological function;
  ○ providing that information signals, including said sensor signals $ST_{i,left}$, $ST_{i,right}$ (i=1, ..., Ns), or parts thereof or data originating therefrom, can be exchanged between the left and right hearing devices and/or forwarded to an auxiliary device.

The method further comprises

- comparing said left and right sensor signals $ST_{i,left}$, $ST_{i,right}$ (i=1, ..., $N_S$), or parts thereof, or data originating therefrom, and providing respective comparison signals $CSI_i$ (i=1, ..., Ns) for each of said physiological functions; and
- analyzing said comparison signals $CSI_i$ (i=1, ..., $N_S$) and providing a concluding stroke indicator CSI regarding a risk of stroke of the user depending on said comparison signal(s).

It is intended that some or all of the structural features of the system described above, in the 'detailed description of embodiments' or in the claims can be combined with embodiments of the method, when appropriately substituted by a corresponding process and vice versa. Embodiments of the method have the same advantages as the corresponding systems.

A computer readable medium:

In an aspect, a tangible computer-readable medium storing a computer program comprising program code means for causing a data processing system to perform at least some (such as a majority or all) of the steps of the method described above, in the 'detailed description of embodiments' and in the claims, when said computer program is executed on the data processing system is furthermore provided by the present application.
By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer-readable media. In addition to being stored on a tangible medium, the computer program can also be transmitted via a transmission medium such as a wired or wireless link or a network, e.g. the Internet, and loaded into a data processing system for being executed at a location different from that of the tangible medium.

A computer program:

A computer program (product) comprising instructions which, when the program is executed by a computer, cause the computer to carry out (steps of) the method described above, in the 'detailed description of embodiments' and in the claims is furthermore provided by the present application.

A data processing system:

In an aspect, a data processing system comprising a processor and program code means for causing the processor to perform at least some (such as a majority or all) of the steps of the method described above, in the 'detailed description of embodiments' and in the claims is furthermore provided by the present application.

An APP:

In a further aspect, a non-transitory application, termed an APP, is furthermore provided by the present disclosure. The APP comprises executable instructions configured to be executed on an auxiliary device to implement a user interface for a hearing system described above in the 'detailed description of embodiments', and in the claims. In an embodiment, the APP is configured to run on cellular phone, e.g. a smartphone, or on another portable device allowing communication with said hearing device or said hearing system.

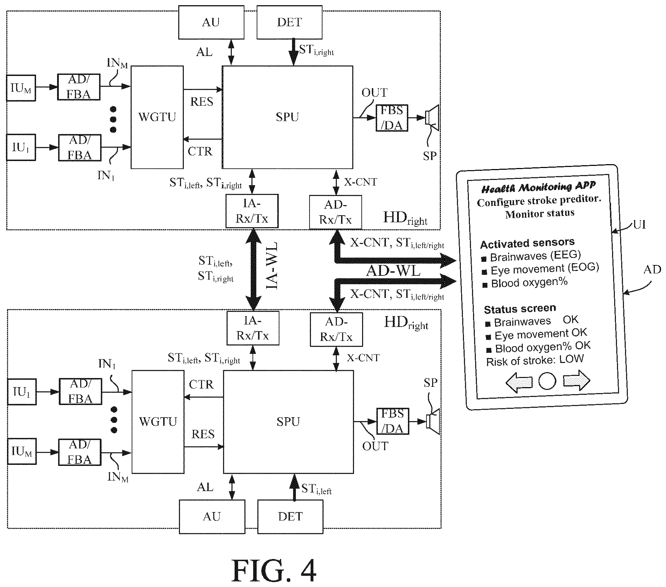

In an embodiment, the hearing system is configured to allow an exchange of configuration data and recorded physiological measures between the auxiliary device and the left and right hearing devices. Thereby functionality of a *Health Monitoring APP* configured to be executed on auxiliary device (AD) in FIG. 4 is enabled. In an embodiment, estimation of a risk of stroke is performed by the APP in the auxiliary device based on sensor data from the left and right hearing devices.

Definitions:

In the present context, a 'hearing device' refers to a device, such as a hearing aid, e.g. a hearing instrument, or an active ear-protection device, or other audio processing device, which is adapted to improve, augment and/or protect the hearing capability of a user by receiving acoustic signals from the user's surroundings, generating corresponding audio signals, possibly modifying the audio signals and providing the possibly modified audio signals as audible signals to at least one of the user's ears. A 'hearing device' further refers to a device such as an earphone or a headset adapted to receive audio signals electronically, possibly modifying the audio signals and providing the possibly modified audio signals as audible signals to at least one of the user's ears. Such audible signals may e.g. be provided in the form of acoustic signals radiated into the user's outer ears, acoustic signals transferred as mechanical vibrations to the user's inner ears through the bone structure of the user's head and/or through parts of the middle ear as well as electric signals transferred directly or indirectly to the cochlear nerve of the user.

The hearing device may be configured to be worn in any known way, e.g. as a unit arranged behind the ear with a tube leading radiated acoustic signals into the ear canal or with an output transducer, e.g. a loudspeaker, arranged close to or in the ear canal, as a unit entirely or partly arranged in the pinna and/or in the ear canal, as a unit, e.g. a vibrator, attached to a fixture implanted into the skull bone, as an attachable, or entirely or partly implanted, unit, etc. The hearing device may comprise a single unit or several units communicating electronically with each other. The loudspeaker may be arranged in a housing together with other components of the hearing device, or may be an external unit in itself (possibly in combination with a flexible guiding element, e.g. a dome-like element).

More generally, a hearing device comprises an input transducer for receiving an acoustic signal from a user's surroundings and providing a corresponding input audio signal and/or a receiver for electronically (i.e. wired or wirelessly) receiving an input audio signal, a (typically configurable) signal processing circuit (e.g. a signal processor, e.g. comprising a configurable (programmable) processor, e.g. a digital signal processor) for processing the input audio signal and an output unit for providing an audible signal to the user in dependence on the processed audio signal. The signal processor may be adapted to process the input signal in the time domain or in a number of frequency bands. In some hearing devices, an amplifier and/or compressor may constitute the signal processing circuit. The signal processing circuit typically comprises one or more (integrated or separate) memory elements for executing programs and/or for storing parameters used (or potentially used) in the processing and/or for storing information relevant for the function of the hearing device and/or for storing information (e.g. processed information, e.g. provided by the signal processing circuit), e.g. for use in connection with an interface to a user and/or an interface to a programming device. In some hearing devices, the output unit may comprise an output transducer, such as e.g. a loudspeaker for providing an air-borne acoustic signal or a vibrator for providing a structure-borne or liquid-borne acoustic signal. In some hearing devices, the output unit may comprise one or more output electrodes for providing electric signals (e.g. a multi-electrode array for electrically stimulating the cochlear nerve).

In some hearing devices, the vibrator may be adapted to provide a structure-borne acoustic signal transcutaneously or percutaneously to the skull bone. In some hearing devices, the vibrator may be implanted in the middle ear and/or in the inner ear. In some hearing devices, the vibrator may be adapted to provide a structure-borne acoustic signal to a middle-ear bone and/or to the cochlea. In some hearing devices, the vibrator may be adapted to provide a liquid-borne acoustic signal to the cochlear liquid, e.g. through the oval window. In some hearing devices, the output electrodes may be implanted in the cochlea or on the inside of the skull bone and may be adapted to provide the electric signals to the hair cells of the cochlea, to one or more hearing nerves, to the auditory brainstem, to the auditory midbrain, to the auditory cortex and/or to other parts of the cerebral cortex.

A hearing device, e.g. a hearing aid, may be adapted to a particular user's needs, e.g. a hearing impairment. A configurable signal processing circuit of the hearing device may be adapted to apply a frequency and level dependent compressive amplification of an input signal. A customized frequency and level dependent gain (amplification or compression) may be determined in a fitting process by a fitting system based on a user's hearing data, e.g. an audiogram, using a fitting rationale (e.g. adapted to speech). The frequency and level dependent gain may e.g. be embodied in processing parameters, e.g. uploaded to the hearing device via an interface to a programming device (fitting system), and used by a processing algorithm executed by the configurable signal processing circuit of the hearing device.

A 'hearing system' refers to a system comprising one or two hearing devices, and a 'binaural hearing system' refers to a system comprising two hearing devices and being adapted to cooperatively provide audible signals to both of the user's ears. Hearing systems or binaural hearing systems may further comprise one or more 'auxiliary devices', which communicate with the hearing device(s) and affect and/or benefit from the function of the hearing device(s). Auxiliary devices may be e.g. remote controls, audio gateway devices, mobile phones (e.g. SmartPhones), or music players. Hearing devices, hearing systems or binaural hearing systems may e.g. be used for compensating for a hearing-impaired person's loss of hearing capability, augmenting or protecting a normal-hearing person's hearing capability and/or conveying electronic audio signals to a person. Hearing devices or hearing systems may e.g. form part of or interact with public-address systems, active ear protection systems, handsfree telephone systems, car audio systems, entertainment (e.g. karaoke) systems, teleconferencing systems, classroom amplification systems, etc.

Embodiments of the disclosure may e.g. be useful in applications such as wearables comprising first and second devices adapted for being mounted on left and right sides of the head of the user, e.g. hearing systems comprising hearables comprising left and right ear pieces, e.g. hearing aids.

BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 shows a scheme for monitoring a risk of stroke of a user according to an embodiment of the present disclosure,

FIG. 2A shows a first embodiment of a hearing system comprising left and right hearing devices, each comprising a number of electrodes for picking up electric signals from a user's body, and

FIG. 2B a second embodiment of a hearing system comprising left and right hearing devices, each comprising a number of electrodes for picking up electric signals from a user's body and exchanging data with an auxiliary device,

FIG. 3 shows an embodiment of a hearing system according to the present disclosure comprising left and right hearing devices and a number of sensors mounted on a spectacle frame, and

FIG. 4 shows an embodiment of a binaural hearing system comprising left and right hearing devices and an auxiliary device in communication with each other according to the present disclosure.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practised without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

The present application relates to the field of hearing devices, e.g. hearing aids.

Strokes result from poor blood flow to the brain that creates brain cell death. Strokes are either ischemic or haemorrhagic. Over 85% of strokes are ischemic. Ischemic strokes are caused by poor blood supply to the brain (often due to a blood clot), while haemorrhagic strokes result from the rupture of a blood vessel, creating bleeding.

Every year over 3 million people worldwide suffer a stroke. Many of the risk factors for a stroke are lifestyle related and include high blood pressure, tobacco smoking, obesity, high blood cholesterol, and diabetes. As the incidence of lifestyle related health problems continue to raise around the world, so does the incidence of strokes.

The location (also known as site of lesion) of a stroke varies. A stroke can occur in the brain cortex, in the cerebellum, in the brainstem, or in the area between the brain and the skull. Symptoms include reduced muscle function, which can be seen with muscle weakness in the arm and face and slurred speech. Most often, the site of lesion is on one side of the head and therefore the symptoms affect only one side of the body (unilateral).

Early detection of stroke is essential to reduce the chances of death and of permanent disability.

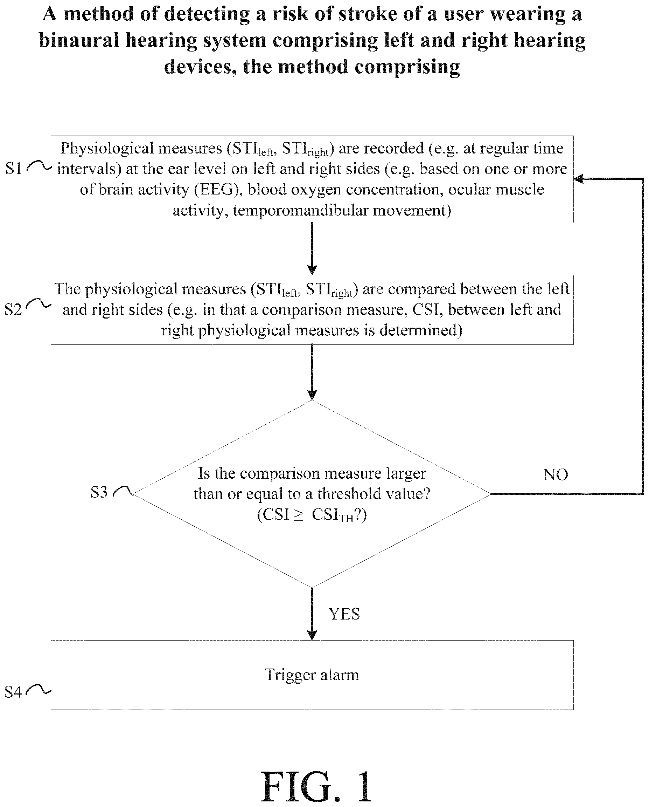

The present disclosure proposes to use bilateral ear-level devices (e.g. hearing devices) to monitor physiological function(s) and to detect any unilateral changes in physiological function. A hearing system according to the present disclosure may comprise the following components (cf. e.g. FIG. 1):

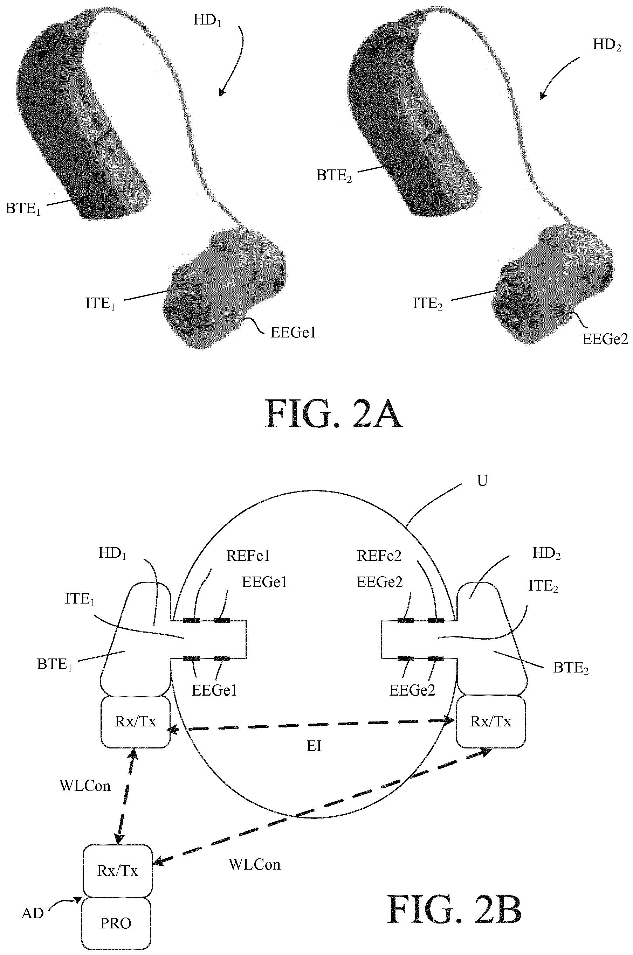

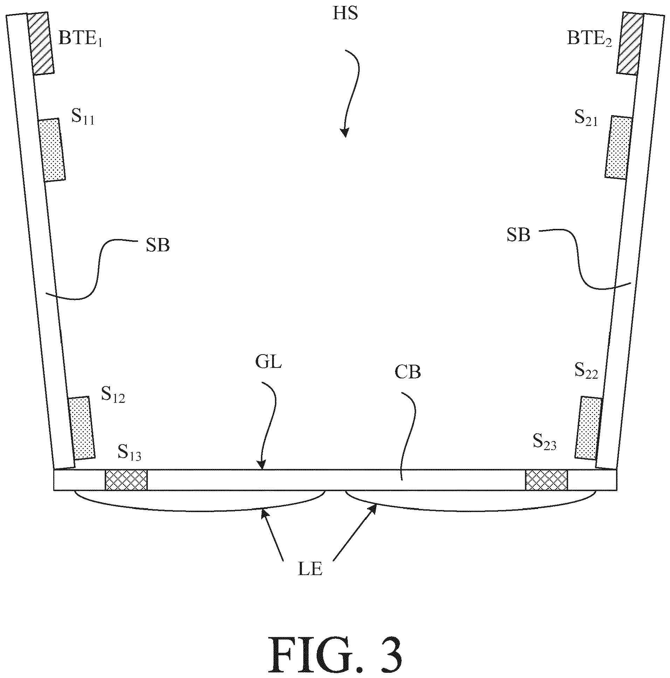

1. **An ear-level device:** This is a hearing device, such as a hearing aid, e.g. an air conduction based hearing instrument, a cochlear implant type hearing instrument, or a bone-conducting hearing system, a 'hearable' / personal sound amplifying product intended for people without hearing loss, or any other ear-level device (cf. e.g. FIG. 2A, 2B). The device could also include exogenous sensors, for example located on smart glasses (e.g. pupillometry, cf. e.g. FIG. 3) or headbands (e.g. electroencephalography with better precision), to take the physiological measures.

2. **A physiological change monitor:** We propose to monitor physiological functions with ear-level measures, with focus on the detection of any *unilateral changes* that would warn about a possible stroke (e.g. including EEG and/or EOG measurements monitoring brain and/or eye movement activity of the user, cf. e.g. FIG. 2A, 2B). A unique physiological measure may be used, or the system may combine several physiological measures for best accuracy (high sensitivity and specificity; few false positives and false negatives).

3. **A data analyzer:** The physiological measures are compared between the sides (difference right-left) for the identification of any unilateral changes. Artificial intelligence, big data, and deep neural networks allow for data analysis and for thresholds in differences allowed that are informed by normative data that is individualised for each user for best accuracy during a calibration period. Thereby initial threshold values can be conservative but adjusted over time based on the values collected for a specific user over the calibration period. In an embodiment, the hearing system is configured to provide that the calibration period occurs automatically when using the devices for the first time - and to allow for manual initiation later on, as needed. If unilateral physiological changes are identified, a risk of eminent stroke is estimated, and an alarm or an optional alert, feedback, and advice system may be triggered. In an embodiment, the hearing system is configured to differentiate an eminent risk of stroke from a general risk factor for stroke, e.g. due to a low level of physical activity or poor diet, etc.

The hearing system may further comprise the following component:

4. **Alert, feedback, and advice system:** An alert is e.g. sent to the emergency services (e.g. telephone 112 in Europe or 911 in North America), to the closest nurse ward for hospitalized patients, or to a family member. The alert can include a text message, alerting the recipient that the user is suffering a stroke without relying on the user having to give verbal commands whilst suffering the stroke. Alert settings are selected by the user. When the alert is sent, the device may be configured to provide feedback (e.g. "help is on its way") and advice (e.g. Dos and Don'ts while waiting for an ambulance, etc.) to its user and care assistants. This functionality may e.g. be implemented in an auxiliary device, e.g. a remote control, e.g. implemented as an APP of a smartphone or similar device (cf. e.g. FIG. 4). In an embodiment, the hearing system is configured to alert directly a contact defined as "In case of emergency" in a mobile device, e.g. in Apple Health for Apple devices.

FIG. 1 shows a scheme for monitoring a risk of stroke of a user according to an embodiment of the present disclosure. FIG. 1 illustrates basic steps of an embodiment of the present disclosure in the form of a method of estimating a risk of stroke of a user wearing a binaural hearing system comprising left and right hearing devices. The method comprises

S1. Physiological measures are taken at the ear level. In an embodiment. physiological measures ($ST_{left}$, $STI_{right}$) are recorded (e.g. at regular time intervals or at predetermined points in time, or when predetermined events are occurring, such as abnormal physical activity) at the ear level on left and right sides (e.g. based on one or more of brain activity (EEG), blood oxygen concentration, ocular muscle activity, temporomandibular movement).

S2. The physiological measures ($STI_{left}$, $STI_{right}$) are compared between the left and right sides (e.g. in that a comparison measure, CSI, between left and right physiological measures is determined).

S3. If unilateral physiological changes are measured, a risk of stroke is detected. Is the comparison measure larger than or equal to a threshold value (i.e. is $CSI \geq CSI_{TH}$?)? If NO, return to S1. If Yes, go to S4.

S4. An alarm is triggered (e.g. in that the user and/or a caretaker is informed about a risk of stroke, e.g. via the hearing devices and or via a remote control or display device (e.g. a smartphone), e.g. via a network, e.g. to a predefined receiver (e.g. a family member or caretaker or alarm unit), cf. also point 4) above ('Alert, feedback, and advice system').

FIG. 2A shows a first embodiment of a hearing system comprising left and right hearing devices, each comprising a number of electrodes for picking up electric signals from a user's body. FIG. 2A shows a pair of behind the ear (BTE) hearing devices with EEG electrodes on the surface of an in-the-ear (ITE) part, e.g. an ear mould. The electrodes may form part of respective sensors of the left and right hearing devices for picking up signals from the user's brain (e.g. EEG potentials) and/or for monitoring eye movement (e.g. EOG potentials or eye muscle activity). In an embodiment, the sensors may comprise electric potential sensor(s) (EPS) with integrated circuits (EPIC) in the ear canal. In an embodiment, the electrodes are conventional (e.g. dry or wet) electrodes for establishing a direct electric contact between skin and electrode. The electrodes may e.g. be located on the surface of in-the-ear moulds (as illustrated in FIG. 2A), in domes for positioning an ITE-part in the ear canal, or integrated in a BTE-part.

FIG. 2B shows an embodiment of a hearing system comprising left and right hearing devices, each comprising a number of EEG and reference electrodes for picking up electric signals from a user's body and exchanging data with an auxiliary device. In the embodiment of FIG. 2B, the first and second (right and left) hearing devices ($HD_1$, $HD_2$) comprises BTE parts (*BTE1, BTE2*), respectively, adapted for being located at or behind an ear of a user (U). Further, the first and second hearing devices ($HD_1$, $HD_2$) each comprises EEG-electrodes (*EEGe1, EEGe2*), and a reference electrode (*REFe1, REFe2*), respectively, arranged on the outer surface of the ITE parts (*ITE1, ITE2*) adapted for being located fully or partially in an ear canal of the user (U). When the first and second hearing devices are operationally mounted on the user, the electrodes of the ear pieces are positioned to have electrical contact with the skin of the user to enable the sensing of body signals (e.g. brainwave signals). In the embodiment of FIG. 2B, three EEG-electrodes (*EEGe1, EEGe2*) are shown on each ITE-part, but more or less may be present in practice depending on the application. Further a reference electrode (*REFe1, REFe2*) is shown on each ITE part. Thereby the reference voltage ($V_{REF2}$) picked up by the reference electrode (*REFe2*) of the second ITE part (*ITE2*) can be used as a reference voltage for the EEG potentials ($V_{EEG1i}$, $i=1, 2, 3$) picked up by the (three) EEG electrodes (*EEGe1*) of the first ITE part (*ITE1*), and vice versa. In an embodiment, the first and second hearing devices provides a binaural hearing system. The reference voltages ($V_{REF1}$, $V_{REF2}$) may be transmitted from one part to the other ($HD_1$ <-> $HD_2$) via electric interface *EI* (cf. e.g. US20160081623A1, and optionally via an auxiliary device (*AD*), e.g. a remote control device, e.g. a smartphone). The auxiliary device (*AD*) may e.g. be configured to process body signals, e.g. EEG-signals, (cf. processing unit (*PRO*) and optionally performing other processing tasks related to the hearing system) and/or providing a user interface for the hearing system (cf. e.g. FIG. 5). Each of the first and second hearing devices ($HD_1$, $HD_2$) and the auxiliary device (*PRO*) comprises antenna and transceiver circuitry (*Rx/Tx*) configured to establish a wireless link (*WLCon*) to each other. The two sets of EEG-signal voltage differences ($\Delta V_{EEG1}$, $\Delta V_{EEG2}$) can be used separately in each of the respective first and second hearing devices ($HD_1$, $HD_2$) (e.g. to control processing of an input audio signal) or combined in one of the hearing devices and/or in the auxiliary device (*PRO,* e.g. for display and/or further processing), e.g. to provide comparison signals CSI for one or more physiological functions, e.g. brain activity (e.g. (ear) EEG signals) and/or eye activity (e.g. (ear) EOG signals), as proposed in the present disclosure.

FIG. 3 shows an embodiment of a hearing system according to the present disclosure comprising left and right hearing devices and a number of sensors mounted on a spectacle frame. The hearing system (HS) comprises a number of sensors $S_{1i}$, $S_{2i}$ ($i=1, ..., N_S$) associated with (e.g. forming part of or connected to) left and right hearing devices ($HD_1$, $HD_2$), respectively. The first, second and third sensors $S_{11}$, $S_{12}$, $S_{13}$ and $S_{21}$, $S_{22}$, $S_{23}$ are mounted on a spectacle frame of the glasses (GL). In the embodiment of FIG. 3, sensors $S_{11}$, $S_{12}$ and $S_{21}$, $S_{22}$ are mounted on the respective sidebars ($SB_1$ and $SB_2$), whereas sensors $S_{13}$ and $S_{23}$ are mounted on the cross bar (CB) having hinged connections to the right and left side bars ($SB_1$ and $SB_2$). Glasses or lenses (LE) of the spectacles are mounted on the cross bar (CB). The left and right hearing devices ($HD_1$, $HD_2$) comprises respective BTE-parts ($BTE_1$, $BTE_2$), and may e.g. further comprise respective ITE-parts ($ITE_1$, $ITE_2$). The ITE-parts may e.g. comprise electrodes for picking up body signals from the user, e.g. forming part of sensors $S_{1i}$, $S_{2i}$ ($i=1, ..., N_S$) for monitoring physiological functions of the user, e.g. brain activity or eye movement activity or temperature (cf. e.g. FIG. 2A, 2B). The sensors mounted on the spectacle frame may e.g. comprise one or more of an eye camera (e.g. for monitoring pupillometry), a blood sensor for measuring oxygen in the blood, a sensor for monitoring Temporomandibular joint (TMJ) movement and/or neck muscle (sternocleidomastoid) activity (e.g. a radar sensor).

FIG. 4 shows an embodiment of a binaural hearing system comprising left and right hearing devices and an auxiliary device in communication with each other according to the present disclosure. FIG. 4 shows an embodiment of a binaural hearing system comprising left and right hearing devices (HD$_{left}$, HD$_{right}$) and an auxiliary device (AD) in communication with each other according to the present disclosure. The left and right hearing devices are adapted for being located at or in left and right ears and/or for fully or partially being implanted in the head at left and right ears of a user. The left and right hearing devices and the auxiliary device (e.g. a separate processing or relaying device, e.g. a smartphone or the like) are configured to allow an exchange of data between them (cf. links IA-WL and AD-WL in FIG. 4), including exchanging the (possibly amplified and/or digitized) output from electronic circuitry coupled to the sensor part (DET, e.g. a number of health sensors, e.g. bio sensors, e.g. comprising electrodes or sensors for picking up signals from the body of the user), or signals based thereon, e.g. EarEEG and/or EarEOG signals, which are fully or partially picked up by the respective left and right hearing devices. The binaural hearing system comprises a user interface (UI) fully or partially implemented in the auxiliary device (AD), e.g. as an APP, cf. *Health Monitoring APP* screen of the auxiliary device (AD) in FIG. 4. In the embodiment, of FIG. 4, at least one of the hearing devices and the auxiliary device comprises an alarm unit (AU) for initiating and/or providing an alarm to the user and/or to other systems or persons of relevance to the user and/or to the handling of a stroke (cf. signal AL). The issue of an alarm may be initiated in the auxiliary device, and e.g. presented on the user interface (UI), e.g. in the status screen (cf. *Health Monitoring APP* in FIG. 4).

The left and right hearing devices each comprise a forward path between M input units IU$_i$, $i$=1, ..., M (each comprising e.g. an input transducer, such as a microphone or a microphone system and/or a direct electric input (e.g. a wireless receiver)) and an output unit (SP), e.g. an output transducer, here a loudspeaker. A beamformer or selector (WGTU) and a signal processor (SPU) is located in the forward path. In an embodiment, the signal processor is adapted to provide a frequency dependent gain according to a user's particular needs. In the embodiment of FIG. 4, the forward path comprises appropriate analogue to digital converters and analysis filter banks (AD/FBA) to provide input signals IN$_1$, ..., IN$_M$ (and to allow signal processing to be conducted) in frequency sub-bands (in the (time-) frequency domain). In another embodiment, some or all signal processing of the forward path is conducted in the time domain. The weighting unit (beamformer or mixer or selector) (WGTU) provides beamformed or mixed or selected signal RES based on one or more of the input signals IN$_1$, ..., IN$_M$. The function of the weighting unit (WGTU) is controlled via the signal processor (SPU), cf. signal CTR, e.g. influenced by the user interface (signal X-CNT). The forward path further comprises a synthesis filter bank and appropriate digital to analogue converter (FBS/DA) to prepare the processed frequency sub-band signals OUT from the signal processor (SPU) as an analogue time domain signal for presentation to a user via the output transducer (loudspeaker) (SP).

The *Health Monitoring APP* of the auxiliary device (AD) in FIG. 4 is e.g. embodied in an APP of a smartphone. From the screen shown in FIG: 4, a stroke predictor can be configured and the results monitored (and configuration data can be transferred to the hearing device(s) to perform physiological measure recording based thereon and results can be transferred to the auxiliary device and availed to the *Health Monitoring APP* for possible analysis and display, cf. e.g. FIG. 1). Appropriate sensors for contributing to the (stroke related) health monitoring provided by the hearing system can be selected by ticking selection boxes to the left of the available sensors. In the example of FIG. 4, all three sensors *Brainwaves (EEG), Eye movement (EOG)* and *Bloodoxygen%* are selected (as indicated by solid square 'tick-box' ■).

A *Status* box is provided below the *Activated sensors* box. The *Status* box is configured to provide feedback to the user (or a care assistant wearing the auxiliary device). In the situation exemplified in FIG. 4, the status of all three sensors is OK (i.e. no sign of stroke or an upcoming stroke based on comparison signals CSI$_i$ for the three selected sensors), and a concluding value of the stroke indicator CSI regarding a risk of stroke of the user is presented as 'Risk of stroke: LOW'.

Example:

The below table lists some examples of physiological functions and corresponding (possible) physiological measurement techniques for the estimation of stroke.

| Physiological function (focus is on detection of any unilateral changes compared to individual baseline) | Physiological measure(s) |
|---|---|
| *Brain activity* | • *Electroencephalography at ear level* |
| *Blood oxygen saturation* | • *Pulse oximetry at ear level* |

(continued)

| Physiological function (focus is on detection of any unilateral changes compared to individual baseline) | Physiological measure(s) |
|---|---|
| Ocular muscle activity | • Electrooculography at ear level<br>• Pupillometry also possible, but not at ear level: could be combined with smart glasses<br>• Video-oculography |
| Temporomandibular joint (TMJ) movement and neck muscle (sternocleidomastoid) activity | • Acoustics of ear canal based on feedback (to monitor changes in ear canal shape because of TMJ movement)<br>• Radar (to monitor TMJ movement and neck muscle activity)<br>• Electromyography at ear level |

Video-oculography represents a measure that is showing promise for early detection of stroke, see e.g. the study of [Toker et al.; 2013]. The study used L/R Vestibulo-ocular reflex (VOR) asymmetry to have a 100% accuracy in detecting stroke.

The present disclosure is exemplified by the estimation of a risk of an upcoming stroke of a user wearing a hearing system comprising health monitoring sensors. Other physiological events than stroke that can be associated with an asymmetric behaviour of physiological parameters detectable at the head (e.g. at the left and right ears) of a user may be correspondingly identified, e.g. epilepsy or other illnesses related to the central nervous system.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

REFERENCES

- [van Putten & Tavy; 2012] Michel J.A.M. van Putten and Dénes L.J. Tavy, Continuous Quantitative EEG Monitoring in Hemispheric Stroke Patients Using the Brain Symmetry Index, Stroke 2004, 35:2489-2492: originally published online October 7, 2004
- EP3035710A2 (OTICON) 22.06.2016
- US20150319542A1 (OTICON) 05-11-2015

- US20160081623A1 (OTICON) 24.03.2016
- [Toker et al.; 2013] Newman-Toker, D. E., Tehrani, A. S. S., Mantokoudis, G., Pula, J. H., Guede, C. I., Kerber, K. A., Ari Blitz, Sarah H. Ying, Yu-Hsiang Hsieh, Richard E. Rothman, Daniel F. Hanley, David S. Zee, Jorge C. Kattah,. Quantitative video-oculography to help diagnose stroke in acute vertigo and dizziness, Stroke, 2013, 44(4), 1158-1161.

**Claims**

1. A binaural hearing system comprising

    • left and right hearing devices, e.g. hearing aids, adapted for being worn at or in left and right ears, respectively, of a user, or for being fully or partially implanted in the head at the left and right ears, respectively, of the user, each of the left and right hearing devices comprising

        ◦ A number Ns of different sensors $S_i$ (i=1, ..., Ns), each sensor being configured to monitor a physiological function of the user and providing respective left and right sensor signals $ST_{i,left}$, $ST_{i,right}$ (i=1, ..., $N_S$), indicative of the state of the physiological function in question;
        ◦ Antenna and transceiver circuitry to provide that information signals, including said sensor signals $ST_{i,left}$, $ST_{i,right}$, or parts thereof or data originating therefrom, can be exchanged between the left and right hearing devices and/or forwarded to an auxiliary device;

    wherein the hearing system further comprises

        • A comparison unit for comparing said left and right sensor signals $ST_{i,left}$, $ST_{i,right}$ (i=1, ..., Ns), or parts thereof, or data originating therefrom, and providing respective comparison signals $CSI_i$ (i=1, ..., Ns) for each of said physiological functions; and
        • An analysis unit for analyzing said comparison signals $CSI_i$ (i=1, ..., $N_S$) and providing a concluding stroke indicator CSI regarding a risk of stroke of the user depending on said comparison signal(s).

2. A binaural hearing system according to claim 1 wherein at least one of said number of sensors comprises an electrode for picking up electric signals of the body.

3. A binaural hearing system according to claim 1 or 2 wherein at least one of said sensors is configured to measure brain activity, e.g. to pick up signals from the user's brain, e.g. EEG-potentials.

4. A binaural hearing system according to any one of claims 1-3 wherein at least one of said sensors is configured to measure ocular muscle activity, e.g. using electrooculography (EOG).

5. A binaural hearing system according to any one of claims 1-4 wherein at least one of said number of sensors is configured to measure oxygen saturation of the user's blood, e.g. using pulse oxiometry.

6. A binaural hearing system according to any one of claims 1-5 wherein at least one of said number of sensors is configured to monitor jaw activity or neck muscle activity.

7. A binaural hearing system according to claim 6 wherein the sensor configured to monitor TMJ activity or neck muscle activity comprises a radar sensor.

8. A binaural hearing system according to any one of claims 1-7 wherein said number N of different sensors S; (i=1, ..., N) comprises a first sensor comprising an electrode for picking up electric signals of the body, e.g. EEG signals and/or EOG signals, and at least one additional sensor.

9. A binaural hearing system according to claims 8 wherein said concluding stroke indicator CSI is based on a predefined conditional criterion regarding said comparison signals $CSI_i$ or sensor stroke indicator $SSI_i$ (i=1, ..., $N_S$) of said first sensor and said at least one additional sensor in such a way that said comparison signal or said sensor stroke indicator of the at least one additional sensor is only considered in case a first comparison signal $CSI_{EEG}$ or a first sensor stroke indicator $SSI_{EEG}$ is indicative of a stroke.

10. A binaural hearing system according to any one of claims 1-9 wherein said predefined criterion regarding said resulting status indicators comprises a degree of asymmetry of the left and right status signals $ST_{i,left}$, $ST_{i,right}$ (i=1, ..., N) as reflected in the corresponding comparison signals $CSI_i$ (i=1, ..., N).

11. A binaural hearing system according to any one of claims 1-10 comprising a wireless interface allowing said concluding stroke indicator and/or said alarm to be forwarded to another device, e.g. via a network.

12. A binaural hearing system according to any one of claims 1-11 wherein the left and right hearing devices comprises left and right hearing aids, headsets, earphones, ear protection devices or a combinations thereof.

13. A binaural hearing system according to any one of claims 1-12 wherein the left and right hearing devices form part of or are mechanically and/or electrically connected to glasses, a head band, a cap, or any other carrier adapted for being located on the head of the user.

14. A method of detecting a risk of stroke of a user wearing a binaural hearing system comprising left and right hearing devices, e.g. hearing aids, adapted for being worn at or in left and right ears, respectively, of a user, or for being fully or partially implanted in the head at the left and right ears, respectively, of the user, the method comprising

   • In each of the left and right hearing devices

     ◦ providing respective left and right sensor signals $ST_{i,left}$, $ST_{i,right}$ (i=1, ..., Ns) indicative of a state of a physiological function;
     ◦ providing that information signals, including said sensor signals $ST_{i,left}$, $ST_{i,right}$, or parts thereof or data originating therefrom, can be exchanged between the left and right hearing devices and/or forwarded to an auxiliary device,

   wherein the method further comprises

     • comparing said left and right sensor signals $ST_{i,left}$, $ST_{i,right}$ (i=1, ..., Ns), or parts thereof, or data originating therefrom, and providing respective comparison signals $CSI_i$ (i=1, ..., Ns) for each of said physiological functions; and
     • analyzing said comparison signals $CSI_i$ (i=1, ..., $N_S$) and providing a concluding stroke indicator CSI regarding a risk of stroke of the user depending on said comparison signal(s).

15. Use of a binaural hearing system as claimed in any one of claims 1-13.

16. A data processing system comprising a processor and program code means for causing the processor to perform the steps of the method of claim 14.

17. A computer program comprising instructions which, when the program is executed by a computer, cause the computer to carry out the method of any one of claim 14.

**A method of detecting a risk of stroke of a user wearing a binaural hearing system comprising left and right hearing devices, the method comprising**

S1 — Physiological measures ($STI_{left}$, $STI_{right}$) are recorded (e.g. at regular time intervals) at the ear level on left and right sides (e.g. based on one or more of brain activity (EEG), blood oxygen concentration, ocular muscle activity, temporomandibular movement)

S2 — The physiological measures ($STI_{left}$, $STI_{right}$) are compared between the left and right sides (e.g. in that a comparison measure, CSI, between left and right physiological measures is determined)

S3 — Is the comparison measure larger than or equal to a threshold value? ($CSI \geq CSI_{TH}$?)  NO

YES

S4 — Trigger alarm

# FIG. 1

FIG. 2A

FIG. 2B

HS

BTE₁

S₁₁

BTE₂

S₂₁

SB

SB

GL

CB

S₁₂

S₂₂

S₁₃

S₂₃

LE

# FIG. 3

FIG. 4

Europäisches
Patentamt

European
Patent Office

Office européen
des brevets

# EUROPEAN SEARCH REPORT

Application Number

EP 17 16 1519

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | EP 3 035 710 A2 (OTICON AS [DK]) 22 June 2016 (2016-06-22) * paragraphs [0001], [0006], [0008], [0010] * * paragraph [0035] - paragraph [0038] * * paragraphs [0045], [0053], [0057], [0058] * * paragraph [0072] - paragraph [0074] * * paragraphs [0077], [0088] * * paragraph [0104] - paragraph [0119] * * paragraph [0126] - paragraph [0130] * * paragraph [0161] - paragraph [0174] * * paragraphs [0182], [0185], [0194] * * figures 4-6, 9B, 9C * | 1-17 | INV. H04R25/00 A61B5/04 A61B5/00 ADD. A61B5/1455 H04R5/033 |
| Y | US 2011/245707 A1 (CASTLE JAMES SHERMAN [US] ET AL) 6 October 2011 (2011-10-06) * paragraphs [0002], [0033] * * paragraph [0034] - paragraph [0046] * * paragraph [0064] - paragraph [0080] * * paragraph [0083] - paragraph [0086] * * paragraph [0089] - paragraph [0092] * * paragraphs [0101], [0103], [0105] * * paragraph [0106] - paragraph [0108] * * paragraph [0110] - paragraph [0116] * * figures 1,2D,3, 4B, 6-8 * | 1-17 | TECHNICAL FIELDS SEARCHED (IPC) H04R A61B G01N |
| A | US 2007/276270 A1 (TRAN BAO [US]) 29 November 2007 (2007-11-29) * paragraph [0011] - paragraph [0012] * * paragraph [0058] * * paragraph [0313] * * figures 1, 13, 14B * | 4,6,7 | |

-/--

The present search report has been drawn up for all claims

| Place of search | Date of completion of the search | Examiner |
|---|---|---|
| The Hague | 17 August 2017 | Valenzuela, Miriam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 17 16 1519

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/061381 A1 (ATLASENSE BIOMED LTD [IL]; ATLAS DAN [IL]) 21 April 2016 (2016-04-21) * paragraphs [0005], [0007], [0008] * * paragraph [0075] - paragraph [0080] * * paragraph [0090] * * paragraph [0110] * * figures 17-19; 21 * ----- | 9 | |
| A | US 2013/343585 A1 (BENNETT JAMES D [CZ] ET AL) 26 December 2013 (2013-12-26) * paragraph [0003] * * paragraph [0028] - paragraph [0032] * * paragraph [0044] * * paragraph [0077]; figures 1-5,7-9 * ----- | 1-17 | |

TECHNICAL FIELDS
SEARCHED        (IPC)

The present search report has been drawn up for all claims

| Place of search | Date of completion of the search | Examiner |
|---|---|---|
| The Hague | 17 August 2017 | Valenzuela, Miriam |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 16 1519

This annex lists the patent family members relating to the patent documents cited in  the above-mentioned European search report.
The members are as contained in the European Patent Office EDP file on
The European Patent Office is in no way liable for these particulars which are merely given for the purpose of information.

17-08-2017

| Patent document cited in search report | | Publication date | Patent family member(s) | | Publication date |
|---|---|---|---|---|---|
| EP 3035710 | A2 | 22-06-2016 | NONE | | |
| US 2011245707 | A1 | 06-10-2011 | NONE | | |
| US 2007276270 | A1 | 29-11-2007 | US | 2007276270 A1 | 29-11-2007 |
| | | | US | 2009318779 A1 | 24-12-2009 |
| | | | US | 2012330109 A1 | 27-12-2012 |
| | | | US | 2013231574 A1 | 05-09-2013 |
| | | | US | 2014249429 A1 | 04-09-2014 |
| | | | US | 2015359467 A1 | 17-12-2015 |
| WO 2016061381 | A1 | 21-04-2016 | NONE | | |
| US 2013343585 | A1 | 26-12-2013 | US | 2013343584 A1 | 26-12-2013 |
| | | | US | 2013343585 A1 | 26-12-2013 |
| | | | US | 2013344806 A1 | 26-12-2013 |
| | | | US | 2016037288 A1 | 04-02-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 16162760 A
- EP 3035710 A2
- US 20150319542 A1
- US 20160081623 A1

### Non-patent literature cited in the description

- **MICHEL J.A.M. VAN PUTTEN ; DÉNES L.J. TAVY.** Continuous Quantitative EEG Monitoring in Hemispheric Stroke Patients Using the Brain Symmetry Index. *Stroke 2004,* 07 October 2004, vol. 35, 2489-2492

- **NEWMAN-TOKER, D. E. ; TEHRANI, A. S. S. ; MANTOKOUDIS, G. ; PULA, J. H. ; GUEDE, C. I. ; KERBER, K. A. ; ARI BLITZ ; SARAH H. YING ; YU-HSIANG HSIEH ; RICHARD E. ROTHMAN.** Quantitative video-oculography to help diagnose stroke in acute vertigo and dizziness. *Stroke,* 2013, vol. 44 (4), 1158-1161